# EUROPEAN PATENT SPECIFICATION

(11) **EP 1 531 691 B1**
(45) Date of publication and mention of the grant of the patent: **14.05.2008**
(21) Application number: 03791485.0
(22) Date of filing: 05.08.2003
(51) Int. Cl.: A23L 1/308, A23L 2/40

(54) **DIETETIC, SOLUBLE, EFFERVESCENT FOOD COMPLEMENT**
DIÄTETISCHES, LÖSLICHES, SPRUDELNDES NAHRUNGSERGÄNZUNGSMITTEL
COMPLEMENT ALIMENTAIRE DIETETIQUE, SOLUBLE ET EFFERVESCENT

(30) Priority: 30.08.2002 ES 201900096
(43) Date of publication of application: 25.05.2005
(73) Proprietor: Romildo Holdings N.V., Curacao (AN)
(72) Inventor: RAKERS, Evert, Curacao (AN)
(74) Representative: Valkonet, Rutger
(86) International application number: PCT/NL2003/000562
(87) International publication number: WO 2004/019702

(56) References cited:
- EP-A- 0 019 675
- WO-A-01/50886
- PATENT ABSTRACTS OF JAPAN vol. 2000, no. 02, 29 February 2000 (2000-02-29) & JP 11 302183 A (FANCL CORP), 2 November 1999 (1999-11-02)
- DATABASE WPI Section Ch, Week 200028 Derwent Publications Ltd., London, GB; Class D13, AN 2000-318295 XP002256007 & BR 9 800 375 A (ARAGAEO CRAVEIRO A), 21 March 2000 (2000-03-21)

## Description

### OBJECT OF THE INVENTION

This specification refers to an application for a Patent of Invention corresponding to a dietetic, soluble, effervescent food complement which appears as a soluble tablet obtained from the fibre of the carapace of crustaceans such dietetic food complement having the quality of sucking the fats eaten catching and retaining them, preventing that they are absorbed by the organism, being thereafter disposed of in a normal way by evacuation.

The evacuation allows the quick action of the fibre of the carapace of crustaceans in its progress.

The invention is designed as a food complement for persons following a special diet to lose weight.

### FIELD OF THE INVENTION

This invention is applied within the industry engaged in manufacturing dietetic and food products.

### BACKGROUND OF THE INVENTION

Japanese Application JP 11302183 discloses a composition in form of a tablet comprising chitosan from shells of crustaceans, a source of calcium and an organic acid. However, the composition is not effervescent.

### DESCRIPTION OF THE INVENTION

The dietetic, soluble, effervescent food complement proposed by the invention is made as a product recommended for helping to lose weight and eliminate fats, acting at same time as a support for persons following a special diet.

Concretely, the dietetic, soluble, effervescent food complement object of this invention is constituted from weighing and collecting the raw material for mixing it then in a mixer.

Then, the soluble effervescent tablets are compressed.

The manufacture or production of the product is carried out meeting HACCP and ISO standards.

### PREFERRED EMBODIMENT OF THE INVENTION

The dietetic food complement which is proposed is constituted from the fibre of the carapace of crustaceans that jointly with other raw materials must be weighed and mixed within the suitable container depending on the formula and/or mixing instructions.

Then, the raw materials are put together and introduced in the mixer, a mixing process being carried out during 7 minutes after which the mixer must be emptied and cleaned.

Then, further to withdrawing the product from the mixer, the soluble effervescent tablets are pressed and after having checked their physical characteristics, colorimetry and state, they are introduced in containers provided with due stoppers.

It must be clearly stated that to each effervescent tablet two very important raw materials are incorporated, namely nitric acid and sodium hydrogen carbonate.

The two ingredients acting as raw materials react in collaboration with water and thereafter the effervescent cycle is obtained while the rest of the ingredients are dissolved by themselves.

Concretely, the ingredients of the effervescent tablet are as follows:
- Fibre of carapace of crustacean 500 mg
- Sorbitol 250 mg
- Ascorbic acid 65 mg
- Citric acid 2,133.6 mg
- Sodium hydrogen carbonate 1,420.0 mg
- Lemon flavouring 60 mg
- Sweetener (aspartame) 32 mg
- Dextrose anhydrate 43 mg
- Sodium sacharin 5 mg
- Riboflavin 6-sodium phosphate 0.4 mg

## Claims

1. Dietetic, soluble, effervescent food complement of those designed for its use as a product eliminating fats, helping to lose weight and assisting the persons who are following a special diet, **characterized in that** it is constituted from the fibre of the carapace of crustaceans, having the shape of a soluble and effervescent tablet, being produced from weighing and mixing the raw materials constituting it in a container, to then introduce them in a mixer during 7 minutes and then introducing such product obtained in a press for transforming it in soluble and effervescent tablets formed by the fibre of the carapace of crustaceans, sorbitol, ascorbic acid, citric acid, sodium hydrogen carbonate, lemon flavouring, sweetener, dextrose anhydrate, sodium sacharin, riboflavin.

2. Dietetic, soluble, effervescent food complement according to the first claim, **characterized in that** the fibre of the carapace of the crustacean is incorporated in an amount of 500 mg in each tablet.

3. Dietetic, soluble, effervescent food complement according to the first claim, **characterized in that** the sorbitol is incorporated in an amount of 250 mg in each tablet.

4. Dietetic, soluble, effervescent food complement according to the first claim, **characterized in that** the ascorbic acid is incorporated in an amount of 65 mg in each tablet.

5. Dietetic, soluble, effervescent food complement according to the first claim, **characterized in that** the citric acid is incorporated in an amount of 2,133.6 mg in each tablet.

6. Dietetic, soluble, effervescent food complement according to the first claim, **characterized in that** the sodium hydrogen carbonate is incorporated in an amount of 1,420.0 mg in each tablet.

7. Dietetic, soluble, effervescent food complement according to the first claim, **characterized in that** the lemon flavouring is incorporated in an amount of 60 mg in each tablet.

8. Dietetic, soluble, effervescent food complement according to the first claim, **characterized in that** the sweetener, in this case aspartame, is incorporated in an amount of 32 mg in each tablet.

9. Dietetic, soluble, effervescent food complement according to the first claim, **characterized in that** the dextrose anhydrate is incorporated in an amount of 34 mg in each tablet.

10. Dietetic, soluble, effervescent food complement according to the first claim, **characterized in that** the sodium sacharin is incorporated in an amount of 5 mg in each tablet.

11. Dietetic, soluble, effervescent food complement according to the first claim, **characterized in that** the riboflavin-6-sodium phosphate is incorporated in an amount of 0.4 mg in each tablet.

## Patentansprüche

1. Diätetisches, lösliches, sprudelndes Nahrungsergänzungsmittel der zur Verwendung als Fettabsonderungserzeugnis entwickelten Art zur Hilfe bei Gewichtsreduzierung und zur Unterstützung von Personen, die eine spezielle Diät befolgen,
**dadurch gekennzeichnet,**
**dass** es aus der Faser der Schale von Krustazeen besteht, die Form einer löslichen und sprudelnden Tablette hat und **dadurch** hergestellt ist, dass die Rohmaterialien, aus denen es gebildet wird, ausgewogen und in einem Behälter gemischt werden, diese dann für 7 Minuten in einen Mischer eingetragen werden und das so erhaltene Produkt anschließend in eine Presse eingebracht wird, um es zu löslichen und sprudelnden Tabletten bestehend aus der Faser der Schale von Krustazeen, aus Sorbitol, Ascorbinsäure, Zitronensäure, Natriumhydrogencarbonat, Zitronenaroma, Süßmittel, Dextroseanhydrat, Kristallose, Riboflavin auszuformen.

2. Diätetisches, lösliches, sprudelndes Nahrungsergänzungsmittel nach Anspruch 1,
**dadurch gekennzeichnet,**
**dass** die Faser der Schale der Krustazee in einer Menge von 500 mg pro Tablette eingebracht ist.

3. Diätetisches, lösliches, sprudelndes Nahrungsergänzungsmittel nach Anspruch 1,
**dadurch gekennzeichnet,**
**dass** das Sorbitol in einer Menge von 250 mg pro Tablette eingebracht ist.

4. Diätetisches, lösliches, sprudelndes Nahrungsergänzungsmittel nach Anspruch 1,
**dadurch gekennzeichnet,**
**dass** die Ascorbinsäure in einer Menge von 65 mg pro Tablette eingebracht ist.

5. Diätetisches, lösliches, sprudelndes Nahrungsergänzungsmittel nach Anspruch 1,
**dadurch gekennzeichnet,**
**dass** die Zitronensäure in einer Menge von 2133,6 mg pro Tablette eingebracht ist.

6. Diätetisches, lösliches, sprudelndes Nahrungsergänzungsmittel nach Anspruch 1,
**dadurch gekennzeichnet,**
**dass** das Natriumhydrogencarbonat in einer Menge von1420,0 g pro Tablette eingebracht ist.

7. Diätetisches, lösliches, sprudelndes Nahrungsergänzungsmittel nach Anspruch 1,
**dadurch gekennzeichnet,**
**dass** das Zitronenaroma in einer Menge von 60 mg pro Tablette eingebracht ist.

8. Diätetisches, lösliches, sprudelndes Nahrungsergänzungsmittel nach Anspruch 1,
**dadurch gekennzeichnet,**
**dass** das Süßmittel, im vorliegenden Fall Aspartam, in einer Menge von 32 mg pro Tablette eingebracht ist.

9. Diätetisches, lösliches, sprudelndes Nahrungsergänzungsmittel nach Anspruch 1,
**dadurch gekennzeichnet,**
**dass** das Dextroseanhydrat in einer Menge von 34 mg pro Tablette eingebracht ist.

10. Diätetisches, lösliches, sprudelndes Nahrungsergänzungsmittel nach Anspruch 1,
**dadurch gekennzeichnet,**
**dass** das die Kristallose in einer Menge von 5 mg pro Tablette eingebracht ist.

11. Diätetisches, lösliches, sprudelndes Nahrungsergänzungsmittel nach Anspruch 1,
**dadurch gekennzeichnet,**
**dass** das Riboflavin-6-natriumphosphat in einer Menge von 0,4 mg pro Tablette eingebracht ist.

## Revendications

1. Complément alimentaire effervescent, soluble, diététique, de ceux conçus pour être utilisés en tant que produit éliminant les graisses, aidant à perdre du poids et assistant les personnes qui suivent un régime spécial, **caractérisé en ce qu'**il est constitué de fibres provenant de la carapace de crustacés, ayant la forme d'un comprimé soluble et effervescent, produit en pesant et en mélangeant les matières premières qui le constituent dans un conteneur, pour les introduire ensuite dans un mélangeur pendant 7 minutes et ensuite introduire le produit ainsi obtenu dans une presse pour le transformer en comprimés solubles et effervescents, constitués de fibres de la carapace de crustacés, de sorbitol, d'acide ascorbique, d'acide citrique, de bicarbonate de soude, d'arôme de citron, d'édulcorant, d'anhydrate de dextrose, de saccharinate de sodium, de riboflavine.

2. Complément alimentaire effervescent, soluble et diététique selon la première revendication, **caractérisé en ce que** la fibre de carapace de crustacés est incorporée dans une quantité de 500 mg dans chaque comprimé.

3. Complément alimentaire effervescent, soluble et diététique selon la première revendication, **caractérisé en ce que** le sorbitol est incorporé dans une quantité de 250 mg dans chaque comprimé.

4. Complément alimentaire effervescent, soluble et diététique selon la première revendication, **caractérisé en ce que** l'acide ascorbique est incorporé dans une quantité de 65 mg dans chaque comprimé.

5. Complément alimentaire effervescent, soluble, diététique selon la première revendication, **caractérisé en ce que** l'acide citrique est incorporé dans une quantité de 2 133,6 mg dans chaque comprimé.

6. Complément alimentaire effervescent, soluble et diététique selon la première revendication, **caractérisé en ce que** le bicarbonate de soude est incorporé dans une quantité de 1 420,0 mg dans chaque comprimé.

7. Complément alimentaire effervescent, soluble et diététique selon la première revendication, **caractérisé en ce que** l'arôme de citron est incorporé dans une quantité de 60 mg dans chaque comprimé.

8. Complément alimentaire effervescent, soluble et diététique selon la première revendication, **caractérisé en ce que** l'édulcorant, dans ce cas l'aspartame, est incorporé dans une quantité de 32 mg dans chaque comprimé.

9. Complément alimentaire effervescent, soluble et diététique selon la première revendication, **caractérisé en ce que** l'anhydrate de dextrose est incorporé dans une quantité de 34 mg dans chaque comprimé.

10. Complément alimentaire effervescent, soluble et diététique selon la première revendication, **caractérisé en ce que** le saccharinate de sodium est incorporé dans une quantité de 5 mg dans chaque comprimé.

11. Complément alimentaire effervescent, soluble et diététique selon la première revendication, **caractérisé en ce que** le phosphate de riboflavine-6-sodium est incorporé dans une quantité de 0,4 mg dans chaque comprimé.
